(19) Europäisches Patentamt
European Patent Office
Office européen des brevets

(11) Veröffentlichungsnummer: **0 388 873 B1**

(12) **EUROPÄISCHE PATENTSCHRIFT**

(45) Veröffentlichungstag der Patentschrift: **11.05.94**

(51) Int. Cl.[5]: **C07D 251/42**, A01N 47/36

(21) Anmeldenummer: **90105198.7**

(22) Anmeldetag: **20.03.90**

Die Akte enthält technische Angaben, die nach dem Eingang der Anmeldung eingereicht wurden und die nicht in dieser Patentschrift enthalten sind.

(54) **Herbizide [[1,3,5-Triazin-2-yl) aminocarbonyl] aminsulfonyl] benzoesäureester, Verfahren zu ihrer Herstellung und ihre Verwendung.**

(30) Priorität: **21.03.89 DE 3909146**

(43) Veröffentlichungstag der Anmeldung:
**26.09.90 Patentblatt 90/39**

(45) Bekanntmachung des Hinweises auf die Patenterteilung:
**11.05.94 Patentblatt 94/19**

(84) Benannte Vertragsstaaten:
**AT BE CH DE DK ES FR GB GR IT LI LU NL SE**

(56) Entgegenhaltungen:
**EP-A- 0 007 687**
**EP-A- 0 030 138**
**EP-A- 0 057 546**

(73) Patentinhaber: **BASF Aktiengesellschaft**
**Carl-Bosch-Strasse 38**
**D-67063 Ludwigshafen(DE)**

(72) Erfinder: **Mayer, Horst, Dr.**
**Faselwiese 19**
**D-6700 Ludwigshafen(DE)**
Erfinder: **Hamprecht, Gerhard, Dr.**
**Rote-Turm-Strasse 28**
**D-6940 Weinheim(DE)**
Erfinder: **Westphalen, Karl-Otto, Dr.**
**Mausbergweg 58**
**D-6720 Speyer(DE)**
Erfinder: **Wuerzer, Bruno, Dr.**
**Ruediger Strasse 13**
**D-6701 Otterstadt(DE)**

EP 0 388 873 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft [[(1,3,5-Triazin-2-yl)aminocarbonyl]aminosulfonyl]benzoesäureester der allgemeinen Formel I

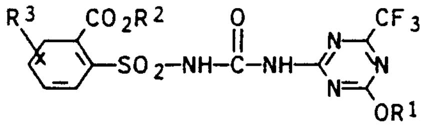

I

in der $R^1$ und $R^2$ eine Methyl- oder Ethylgruppe und $R^3$ Wasserstoff, Fluor oder Chlor bedeutet.

Des weiteren betrifft die Erfindung ein Verfahren zur Herstellung der Verbindungen I sowie ihre Verwendung als herbizide Mittel.

Gegenstand der Erfindung ist ferner ein Verfahren zur Bekämpfung unerwünschten Pflanzenwuchses, das dadurch gekennzeichnet ist, daß man eine herbizid wirksame Menge der Verbindung I oder eines seiner Salze auf die Pflanzen und/oder deren Lebensraum einwirken laßt.

Die EP-A 7687, die EP-A 30 138 und die EP-A 57 546 betreffen Sulfonylharnstoffe mit herbizider Wirkung, deren allgemeine Formel die eingangs definierten Verbindungen I umfaßt.

Während in EP-A 57 546 als nächstliegende Strukturen nur 1,3,5-Triazin-N-oxid-Derivate I'

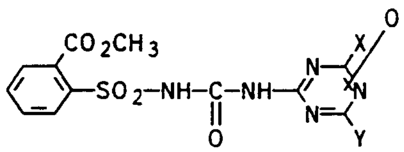

I'

beschrieben sind, in denen die Reste u.a. folgende Bedeutung haben:

X und Y unabhängig voneinander Methyl- oder Methoxygruppe und

O Sauerstoffatom in 1, 3 oder 5-Position des Triazinylrings,

liegt bei der EP-A 30 138 der Schwerpunkt auf der Variation des Carbonsäureesters in 2-Position zur Sulfonamid-Gruppe. Halogenalkylsubstituenten für den Triazin-Teil sind an dieser Stelle nicht vorbeschrieben.

Lediglich in der EP-A 7687 wird ein Triazinylderivat mit Halogenalkylrest beschrieben (I'').

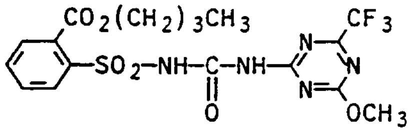

I''

Der Erfindung lag die Aufgabe zugrunde, Sulfonylharnstoffe zu synthetisieren, die gegenüber den bekannten Vertretern dieser Herbizid-Klasse verbesserte Eigenschaften aufweisen.

Entsprechend dieser Aufgabe wurden die eingangs definierten [[(1,3,5-Triazin-2-yl)aminocarbonyl]-aminosulfonyl]benzoesäureester der Formel I gefunden.

Die erfindungsgemäßen Sulfonylharnstoffe der Formel I sind auf verschiedenen Wegen zugänglich, die in der Literatur beschrieben sind. Beispielhaft seien besonders vorteilhafte Wege (A-D) im folgenden näher erläutert.

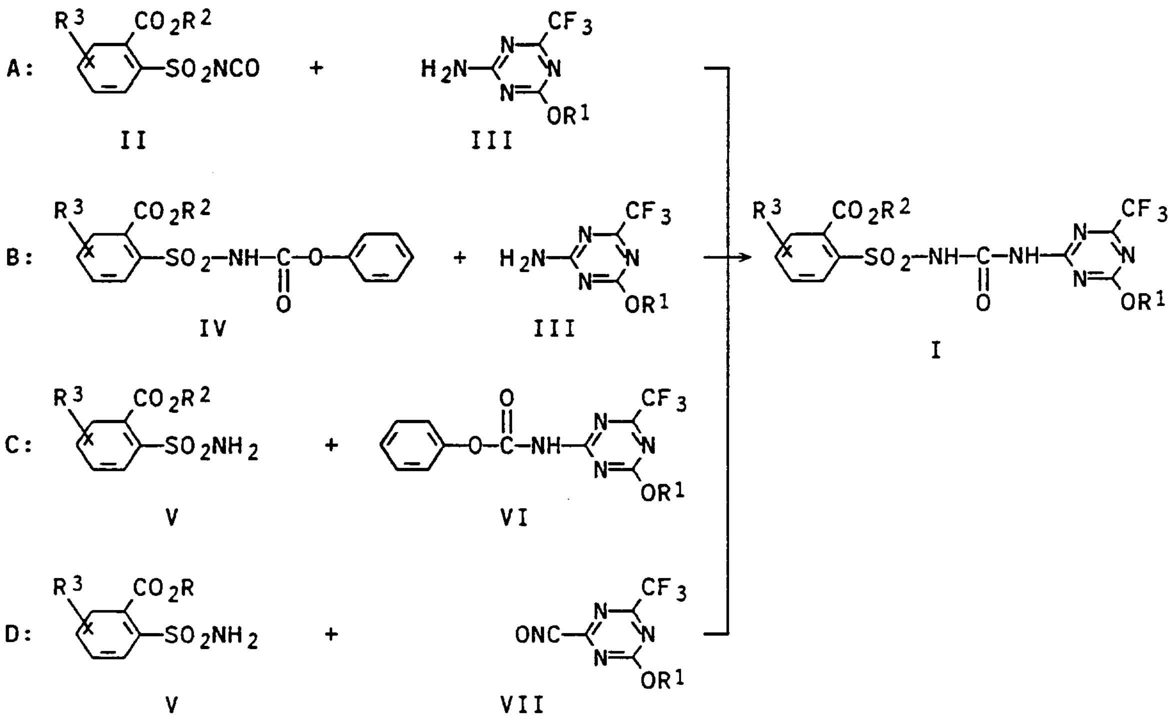

A: II + III
B: IV + III
C: V + VI
D: V + VII
→ I

A: Man setzt ein Sulfonylisocyanat II in an sich bekannter Weise (EP-A-162 723) mit einem Über- oder Unterschuß von 0 bis 20 mol-% eines 2-Amino-1,3,5-triazin-derivats III bei einer Temperatur von 0 bis 120 °C, vorzugsweise 10 bis 100 °C um. Die Reaktion kann unter Normaldruck oder unter Druck (bis 50 bar), vorzugsweise bei 1 bis 5 bar, kontinuierlich oder diskontinuierlich durchgeführt werden.

Zweckmäßigerweise verwendet man für die Umsetzungen unter den jeweiligen Reaktionsbedingungen inerte Lösungs- und Verdünnungsmittel. Als Lösungsmittel kommen beispielsweise Halogenkohlenwasserstoffe, insbesondere Chlorkohlenwasserstoffe, z.B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, m-, p-Dichlorbenzol, o-, p-, m-Dibrombenzol, -o-, m-, p-Chlortoluol, 1,2,4-Trichlorbenzol; Ether, z.B. Ethylpropylether, Methyl-tert.-butylether, n-Butylethylether, Di-n-butylether, Diisobutylether, Diisoamylether, Diisopropylether, Anisol, Phenetol, Cyclohexylmethylether, Diethylether, Ethylenglykoldimethylether, Tetrahydrofuran, Dioxan, Thioanisol, $\beta,\beta'$-Dichlordiethylether; Nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z.B. Heptan, Pinan, Nonan, o-, m-, p-Cymol, Benzinfraktionen innerhalb eines Siedepunktintervalles von 70 bis 190 °C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester, z.B. Ethylacetat, Acetessigester, Isobutylacetat; Amide, z.B. Formamid, Methylformamid, Dimethylformamid; Ketone, z.B. Aceton, Methylethylketon und entsprechende Gemische in Betracht. Zweckmäßig verwendet man das Lösungsmittel in einer Menge von 100 bis 2000 Gew.%, vorzugsweise von 200 bis 700 Gew.%, bezogen auf den Ausgangsstoff II.

Die zur Umsetzung benötigte Verbindung II wird im allgemeinen in etwa äquimolaren Mengen, d.h. mit einem Über- oder Unterschuß von 0 bis 20 %, bezogen auf den jeweiligen Ausgangsstoff III eingesetzt. Man kann den Ausgangsstoff III in einem der vorgenannten Verdünnungsmittel vorlegen und dann den Ausgangsstoff II zugeben.

Zweckmäßigerweise wird das Verfahren zur Herstellung der neuen Verbindungen jedoch so durchgeführt, daß man den Ausgangsstoff II, gegebenenfalls in einem der vorgenannten Verdünnungsmittel, vorlegt und dann den Ausgangsstoff III zugibt.

Zur Beendigung der Umsetzung rührt man nach der Zugabe der Komponenten noch 20 Minuten

bis 24 Stunden bei 0 bis 120 °C, vorzugsweise 10 bis 100 °C, insbesondere 10 bis 100 °C, nach.
Als Reaktionsbeschleuniger kann man vorteilhafterweise ein tertiäres Amin, z.B. Pyridin, α,β,γ-Picolin, 2,4-, 2,6-Lutidin, 2,4,6-Collidin, p-Dimethylaminopyridin, Trimethylamin, Triethylamin, Tri(n-propyl)amin, 1,4-Diaza[2,2,2]bicyclooctan [DABCO] oder 1,8-Diazabicylco]5,4,0]-undec-7-en in einer Menge von 0.01 bis 1 Mol pro Mol Ausgangsstoff II verwenden.
Aus dem Reaktionsgemisch wird der Endstoff I in üblicher Weise, z.B. nach Abdestillieren von Lösungsmittel oder direkt durch Absaugen isoliert. Der verbleibende Rückstand kann noch mit Wasser bzw. verdünnter Säure zur Entfernung basischer Verunreinigungen gewaschen werden. Man kann jedoch auch den Rückstand in einem mit Wasser nicht mischbaren Lösungsmittel lösen und wie beschrieben waschen. Die gewünschten Endstoffe fallen hierbei in reiner Form an, gegebenenfalls können sie durch Umkristallisation, Rühren in einem organischen Lösungsmittel, das die Verunreinigungen aufnimmt oder Chromatographie gereinigt werden.
Bevorzugt führt man diese Umsetzung in Acetonitril, Methyl-tert.-butylether, Toluol oder Methylenchlorid in Anwesenheit von 0 bis 100 Moläquivalenten, vorzugsweise 0 bis 50 Moläquivalenten eines tertiären Amins wie 1,4-Diazabicyclo-[2,2,2]-octan oder Triethylamin durch.

B: Man setzt ein entsprechendes Sulfonylcarbamat der Formel IV in an sich bekannter Weise (EP-A-120 814, EP-A-101 407) in einem inerten organischen Lösungsmittel bei einer Temperatur zwischen 0 und 120 °C, vorzugsweise 10 bis 100 °C mit einem Über- oder Unterschuß von 0 bis 20 mol-% eines 2-Amino-1,3,5-triazin-derivats III um. Es können hierbei Basen wie tertiäre Amine zugesetzt werden, wodurch die Reaktion beschleunigt und die Produktqualität verbessert wird.
Geeignete Basen hierfür sind z.B. tertiäre Amine wie unter A angegeben, insbesondere Triethylamin oder 1,4-Diazabicyclo[2,2,2]octan, in einer Menge von 0,01 bis 1 Mol pro Ausgangsstoff IV.
Zweckmäßig verwendet man als Lösungsmittel die unter A angegebenen.
Man verwendet das Lösungsmittel in einer Menge von 100 bis 2.000 Gew.%, vorzugsweise von 200 bis 700 Gew.%, bezogen auf den Ausgangsstoff IV.
Die zur Umsetzung benötigte Verbindung IV wird im allgemeinen in etwa äquimolaren Mengen, d.h. mit einem Über- oder Unterschuß von 0 bis 20 %, bezogen auf jeweiligen Ausgangsstoff III eingesetzt. Man kann den Ausgangsstoff IV in einem der vorgenannten Verdünnungsmittel vorlegen und dann den Ausgangsstoff III zugeben.
Man kann jedoch auch den Ausgangsstoff III in einem der genannten Löse- oder Verdünnungsmittel vorlegen und das Sulfonylcarbamat IV zugeben.
In beiden Fällen kann als Katalysator vor oder während der Reaktion eine Base zugesetzt werden.
Aus dem Reaktionsgemisch kann das Endprodukt I in üblicher Weise, wie unter A angegeben, gewonnen werden.

C: Man setzt ein Sulfonamid der Formel V in an sich bekannter Weise (EP-A-141 777 und EP-A-101 670) in einem inerten organischen Lösungsmittel mit einem Über- oder Unterschuß von 0 bis 20 mol-% eines Phenylcarbamats VI bei einer Temperatur von 0 bis 120 °C, vorzugsweise 20 bis 100 °C um. Die Reaktion kann bei Normaldruck oder unter Druck (bis 50 bar), vorzugsweise bei 1 bis 5 bar, kontinuierlich oder diskontinuierlich durchgeführt werden.
Es können hierbei Basen wie tertiäre Amine zugesetzt werden, die die Reaktion beschleunigen und die Produktqualität verbessern. Geeignete Basen sind hierfür die unter A angegebenen, insbesondere Triethylamin, 2,4,6-Collidin, 1,4-Diazabicyclo[2,2,2]octan [DABCO] oder 1,8-Diazabicyclo[5,4,0]undec-7-en (DBU), in einer Menge von 0,01 bis 1 Mol pro Ausgangsstoff V.
Zweckmäßigerweise verwendet man als Löse- oder Verdünnungsmittel die unter A angegebenen.
Man verwendet das Lösungsmittel in einer Menge von 100 bis 2000 Gew.%, vorzugsweise von 200 bis 700 Gew.%, bezogen auf das Edukt V.
Die zur Umsetzung benötigte Verbindung V wird im allgemeinen in etwa äquimolaren Mengen, d.h. mit einem Über- oder Unterschuß von 0 bis 20 %, bezogen auf die jeweiligen Ausgangsstoffe VI) eingesetzt. Man kann den Ausgangsstoff VI in einem der vorgenannten Verdünnungsmittel vorlegen und dann den Ausgangsstoff V zugeben.
Man kann jedoch auch den Ausgangsstoff V in einem der genannten Lösungsmittel vorlegen und dann das Carbamat VI zugeben. In beiden Fällen kann als Katalysator vor oder während der Reaktion eine der genannten Basen zugesetzt werden.
Zur Beendigung der Umsetzung rührt man nach der Zugabe der Komponenten noch 20 Minuten bis 24 Stunden bei 0 bis 120 °C, vorzugsweise 10 bis 100 °C, insbesondere 20 bis 80 °C, nach.
Man isoliert die Sulfonylharnstoffe der Formel I aus dem Reaktionsgemisch mit den üblichen Methoden, wie unter A beschrieben.

D: Man setzt ein Sulfonamid der Formel V in an sich bekannter Weise (EP-A-234 352) in einem

inerten organischen Lösungsmittel mit ungefähr der stöchiometrischen Menge eines Isocyanates VII zu einer Temperatur von 0 bis 150 °C, vorzugsweise 10 bis 100 °C um. Die Reaktion kann bei Normaldruck oder unter Druck (bis 50 bar), vorzugsweise bei 1 bis 5 bar, kontinuierlich oder diskontinuierlich durchgeführt werden.

Es können hierbei vor oder während der Reaktion Basen wie tertiäre Amine zugesetzt werden, die die Reaktionsbeschleuniger und die Produktqualität verbessern. Geeignete Basen sind hierfür die unter A angegebenen, insbesondere Triethylamin oder 2,4,6-Collidin, in einer Menge von 0,01 bis 1 Mol pro Ausgangsstoff V.

Zweckmäßigerweise verwendet man als Lösungsmittel die unter A angegebenen. Man setzt das Lösungsmittel in einer Menge von 100 bis 2000 Gew.%, vorzugsweise von 200 bis 700 Gew.%, bezogen auf das Edukt V.

Die zur Umsetzung benötigte Verbindung V wird im allgemeinen in etwa äquimolaren Mengen (mit einem über- oder Unterschuß von z.B. 0 bis 20 %, bezogen auf die Edukte VII) eingesetzt. Man kann den Ausgangsstoff VII in einem der genannten Verdünnungsmittel vorlegen und dann den Ausgangsstoff V zugeben.

Man kann aber auch das Sulfonamid vorlegen und dann das Isocyanat VII zugeben.

Zur Beendigung der Umsetzung rührt man nach der Zugabe der Komponenten noch 20 Minuten bis 24 Stunden bei 0 bis 120 °C, vorzugsweise 10 bis 100 °C, insbesondere 20 bis 80 °C, nach.

Das Endprodukt I kann aus dem Reaktionsgemisch in der üblichen Weise, wie unter A: beschrieben, gewonnen werden.

Die als Edukte verwendeten Sulfonylisocyanate der Formel II wurden nach oder in Analogie zu Literaturmethoden (z.B. EP-A-7687) gewonnen. Die Sulfonylcarbamate der Formel IV wurden nach oder in Analogie zu an sich bekannten Reaktionen hergestellt (z.B. EP-A-120 814). Man kann jedoch auch Sulfonylisocyanate der Formel II in glatter Reaktion mit Phenol in einem Lösungsmittel wie Ether oder Acetonitril in die Carbamate der Formel IV überführen.

Carbamate der Formel VI sind nach oder in Analogie zu bekannten Umsetzungen (z.B. EP-A-141 777) zugänglich, sie lassen sich aber auch aus den entsprechenden Isocyanaten der Formel VII durch Umsetzung mit Phenol, herstellen.

Die Salze der Verbindungen I sind in an sich bekannter Weise zugänglich (EP-A-304 282, US-A 4,599,412). Man erhält sie durch Deprotonierung der entsprechenden Sulfonylharnstoffe I in Wasser oder einem inerten organischen Lösungsmittel bei Temperaturen von -80 °C bis 120 °C, vorzugsweise 0 °C bis 60 °C in Gegenwart einer Base.

Geeignete Basen sind beispielsweise Alkali- oder Erdalkalimetallhydroxide, -hydride, -oxide oder -alkoholate wie Natrium-, Kalium- und Lithiumhydroxid, Natriummethanolat, -ethanolat und -tert.-butanolat, Natrium- und Calziumhydrid und Calziumoxid.

Als Lösungsmittel kommen beispielsweise neben Wasser auch Alkohole wie Methanol, Ethanol und tert.-Butanol, Ether wie Tetrahydrofuran und Dioxan, Acetonitril, Dimethylformamid, Ketone wie Aceton und Methylethylketon und auch halogenierte Kohlenwasserstoffe in Betracht.

Die Deprotonierung kann bei Normaldruck oder bei Drucken bis 50 bar, vorzugsweise bei Normaldruck bis 5 bar Überdruck durchgeführt werden.

Die als Ausgangsstoffe der Formel V benötigten Sulfonamide lassen sich aus den entsprechenden Anthranilsäureestern durch Meerwein-Reaktion und anschließende Umsetzung mit Ammoniak herstellen (Houben-Weyl, 9, 557f (1955)).

Die Edukte 2-Amino-4-methoxy-6-trifluormethyl-1,3,5-triazin und 2-Amino-4-ethoxy-6-trifluormethyl-1,3,5-triazin sind literaturbekannt (Yakugaku Zasshi 95, 499 (1975)).

Die Verbindungen I bzw. die sie enthaltenden herbiziden Mittel sowie deren umweltverträgliche Salze von Alkalimetallen und Erdalkalimetallen können in Kulturen wie Weizen Schadpflanzen sehr gut bekämpfen, ohne die Kulturpflanzen zu schädigen, ein Effekt, der vor allem auch bei niedrigen Aufwandmengen auftritt. Sie können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen u.a. Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch ZuSatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,1 und 95 Gew.%, vorzugsweise zwischen 0,5 und 90 Gew.%, Wirkstoff.

Beispiele für Formulierungen sind:

I. Man vermischt 90 Gewichtsteile der Verbindung Nr. 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 1 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 2 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 3 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 4 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 5 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 2 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 6 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0 kg/ha, vorzugsweise 0,01 bis 1,0 kg/ha.

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |

| Botanischer Name | Deutscher Name |
|---|---|
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |

| Botanischer Name | Deutscher Name |
|---|---|
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Triazinyl-substituierten Sulfonylharnstoffe der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Phenyloxy- bzw. Heteroaryloxyphenylpropionsäuren sowie deren Salze, Ester und Amide und andere in Betracht.

Außerdem kann es von Nutzen sein, die Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Nachstehend sind Beispiele für die Synthese der Verbindungen I wiedergegeben.

1) 2-[[(4-Trifluormethyl-6-methoxy-1,3,5-triazin-2-yl)aminocarbonyl]aminosulfonyl]benzoesäuremethylester

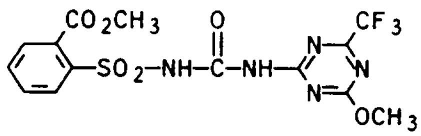

Eine Lösung aus 5,0 g (26 mmol) 2-Amino-4-methoxy-6-trifluormethyl-1,3,5-triazin in 80 ml Acetonitril wurde bei 25 °C mit 9,3 g (39 mmol) 2-Isocyanatosulfonylbenzoesäuremethylester versetzt. Die so erhaltene Mischung wurde 5 Stunden auf 70 °C erwärmt. Nach dem Abkühlen der Reaktionsmischung auf 25 °C wurde mit Methylenchlorid verdünnt und mit 2 n Natronlauge gewaschen. Die wäßrige Phase wurde angesäuert und anschließend mit Methylenchlorid extrahiert. Aus der organischen Phase erhielt man nach der üblichen Aufarbeitung und chromatographischen Reinigung 6,8 g (60 % d.Th.) der gesuchten Verbindung mit Fp. 165-167 °C.

2) 2-[[(4-Trifluormethyl-6-ethoxy-1,3,5-triazin-2-yl)aminocarbonyl]aminosulfonyl]benzoesäuremethylester

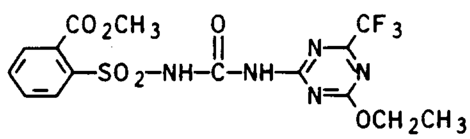

Analog zu den in Beispiel 1 beschriebenen Bedingungen erhielt man aus 5,0 g (24 mmol) 2-Amino-4-ethoxy-6-trifluormethyl-1,3,5-triazin und 7,0 g (29 mmol) 2-Isocyanatosulfonylbenzoesäuremethylester in 80 ml Acetonitril 3,3 g (30 % d.Th.) des gesuchten Produkts mit Fp. 164-168 °C.

3) 4-Chlor-2-[[(4-trifluormethyl-6-methoxy-1,3,5-triazin-2-yl)aminocarbonyl]aminosulfonyl]-benzoesäuremethylester

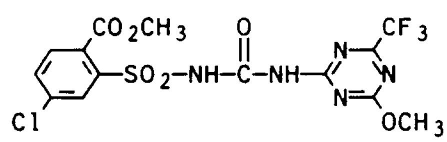

Eine Lösung aus 5,0 g (26 mmol) 2-Amino-4-trifluormethyl-6-methoxytriazin,7,1 g (26 mmol) 4-Chlor-2-isocyanatosulfonylbenzoesäuremethylester und 100 ml Acetonitril wurde 72 h bei 25 °C gerührt. Anschließend wurde das Lösungsmittel bei vermindertem Druck und 40 °C abdestilliert und der dadurch erhaltene Rückstand aus Essigsäureethylester/Ether umkristallisiert. Man erhielt so 6,5 g (53 % d.Th.) der Titelverbindung mit Fp. 148-150 °C.

4) 2-[[(4-Trifluormethyl-6-methoxy-1,3,5-triazin-2-yl)aminocarbonyl]aminosulfonyl]-benzoesäuremethylester-Natriumsalz

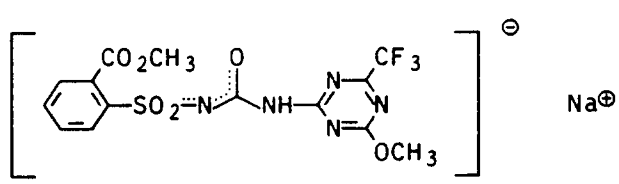

Eine Lösung aus 2,0 g (4,6 mmol) der in Beispiel 1) hergestellten Verbindung, 0,25 g (4,6 mmol)

Natriummethanolat und 50 ml Methanol wurde 1 h bei 25 °C gerührt. Anschließend wurde das Lösungsmittel bei vermindertem Druck und erhöhter Temperatur abdestilliert. Man erhielt das gesuchte Salz in quantitativer Ausbeute, Fp. 175 °C (Zers.).

5) 2-[[(4-Trifluormethyl-6-methoxy-1,3,5-triazin-2-yl)aminocarbonyl]aminosulfonyl]benzolsäuremethylester

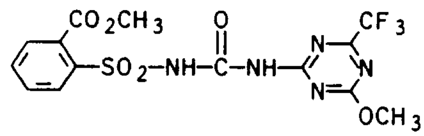

a) 2-Isocyanato-4-methoxy-6-trifluormethyl-1,3,5-triazin Eine Lösung aus 30,0 g 2-Amino-4-methoxy-6-trifluormethyl-1,3,5-triazin (0,154 mol) und 76,2 g Oxalylchlorid (0,6 mol) in 225 ml Toluol wurde 4 Stunden unter Rückfluß gekocht. Die anfängliche starke Gasentwicklung flaute dabei mit der Zeit rasch ab. Nach beendeter Reaktion wurde im Vakuum fraktioniert. Man erhielt 24,8 g des Produkts als bewegliches, farbloses Öl mit Kp. 67-70 °C (0,1 mbar) (73 % d.Th.).

b) Eine Lösung aus 2,2 g 2-Aminosulfonylbenzoesäuremethylester (10 mmol) und 2,2 g 2-Isocyanato-4-methoxy-6-trifluormethyl-1,2,5-triazin in 20 ml Acetonitril wurde 15 Stunden bei 22 bis 25 °C gerührt. Das Reaktionsprodukt wurde, wie unter Punkt 1) beschrieben, gereinigt.

Man erhielt so 2,4 g (55 % d.Th.) des Produktes mit Fp. 165-167 °C.

6. 2-[[(4-Trifluormethyl-6-methoxy-1,3,5-triazin-2-yl)aminocarbonyl]aminosulfonyl]benzoesäuremethylester

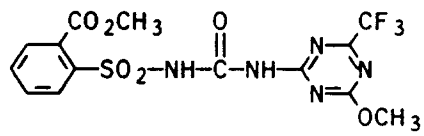

a) 2-Methoxy-4-(phenoxycarbonylamino)-6-trifluormethyl-1,3,5-triazin

Eine Lösung von 100 g 2-Isocyanato-4-methoxy-6-trifluormethyl-1,3,5-triazin (45 mmol) in 50 ml Acetonitril wurde bei 25 °C mit 4,3 g Phenol (45 mmol) versetzt. Nach Zugabe von 30 mg 1,8-Diazabicyclo[2.2.2]octan erwärmte sich das Gemisch auf 40 °C und es trat eine leichte Gelbfärbung auf. Man rührte weitere 15 min und entfernte das Lösungsmittel im Vakuum bei 50 °C. Der fast farblose Rückstand kristallisierte über Nacht. Man erhielt so 14,0 g der Titelverbindung (98 % d.Th.).

$^1$H-NMR-Spektrum ($COCl_3$, 250 MHz, int. TMS, 25 °C), $\delta$ (ppm)): 8.3 br (NH); 7.31 m (2 H ar); 7.24 m (1 H ar); 7.16 m (2 H ar); 4.12 s (3 $OCH_3$).

b) Eine Lösung von 2,3 g 2-Methoxy-4-(phenoxycarbonylamino)-6-trifluormethyl-1,3,5-triazin (7,3 mmol), 1,6 g 2-Methoxycarbonylbenzolsulfonamid (7,3 mmol), 1,1 g 1,8-Diazabicyclo[5.4.0]undec-7-en (7,3 mol) in 10 g Acetonitril wurde 10 min bei 25 °C, 5 min unter Rückfluß, dann 2 h bei 25 °C gerührt. Nach Entfernen des Lösungsmittels im Vakuum bei 50 °C wurde der ölige Rückstand in 200 ml Essigsäureethylester aufgenommen. Die Lösung wurde mit 40 ml 4 N NaOH geschüttelt, dann bei 0 °C mit 50 ml 4 N HCl.

Die organische Phase wurde abgetrennt, mit gesättigter Kochsalzlösung neutral gewaschen, über $Na_2SO_4$ getrocknet und eingeengt. Der ölige Rückstand kristallisierte beim Verrühren mit 200 ml eines Ether-/Pentan-Gemisches (V:V, 1/1). Das Produkt wurde filtriert und im Vakuum bei 40 °C getrocknet.

Man erhielt 1,5 g (47 % d.Th.) der Titelverbindung, identisch mit der nach Weg A: erhaltenen Verbindung.

7. 4-Fluor-2-[[(4-methoxy-6-trifluormethyl-1,3,5-triazin-2-yl)aminocarbonyl]aminosulfonyl]-benzosäuremethylester

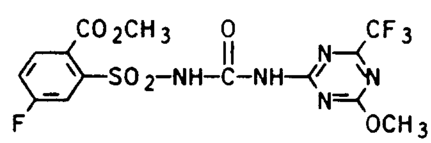

Eine Lösung von 3,7 g 2-Amino-4-methoxy-6-trifluormethyl-1,3,5-triazin(19 mmol) wurde bei 25°C tropfenweise mit 11,0 g einer Lösung aus 4,9 g 4-Fluor-2-isocyanatosulfonylbenzoesäuremethylester (19 mmol) und 1,2-Dichlorethan versetzt. Man rührte 65 h bei 25°C, entfernte die flüchtigen Anteile bei vermindertem Druck bei 60°C und rührte den festen Rückstand kräftig mit 200 ml Diethylether. Das Produkt wurde abfiltriert, mit Ether gewaschen und getrocknet. Man erhielt so 4,4 g der Titelverbindung (51 % d.Th.) mit Fp. 153-157°C.

8) 2-[[(4-Methoxy-6-trifluormethyl-1,3,5-triazin-2-yl)aminocarbonyl]aminosulfonyl]benzoesäuremethylester

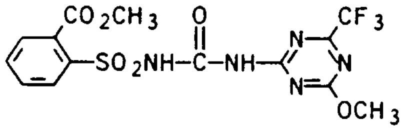

Eine Lösung aus 366,2 g 2-Isocyanatosulfonylbenzoesäuremethylester (1,52 mol) in 280 ml Acetonitril wurde bei 25°C mit 294.5 g 2-Amino-4-methoxy-6-trifluormethyl-1,3,5-triazin (1,52 mol) versetzt. Man rührte 15 h bei dieser Temperatur.

Der gebildete Niederschlag wurde abgesaugt, mit wenig Acetonitril gewaschen und 3 mal mit je 300 ml eines Diethylether-/Hexan-Gemisches (v:v, 1:1) verrührt. Der verbliebene Rückstand wurde isoliert und getrocknet. Man erhielt so 432,0 g (65 % d.Th.) der Titelverbindung (Fp. 165-167°C). Die Mutterlauge wurde auf die Hälfte des Volumens eingeengt und bei 25°C 16 Stunden gelagert. Der gebildete Niederschlag wurde isoliert, 3 mal mit je 500 ml eines Diethylether-/Hexangemisches (v:v, 1:1) gerührt, erneut isoliert und getrocknet. Man erhielt so weitere 92,5 g der Titelverbindung (14 % d.Th.) (Fp. 165-167°C).

9) 2-[[(4-Trifluormethyl-6-methoxy-1,3,5-triazin-2-yl)aminocarbonyl]aminosulfonyl]-benzoesäuremethylester-Calziumsalz

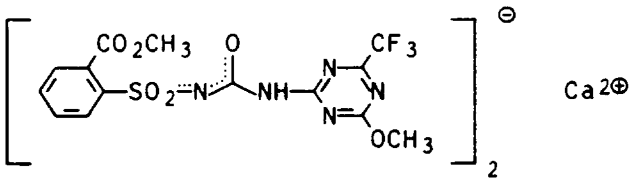

Eine Lösung aus 2,0 g (4,6 mmol) der in Beispiel 1) hergestellten Verbindung, 0,20 g (4,8 mmol) Calziumhydrid und 50 ml Methanol wurde 10 h bei 25°C gerührt. Aus der sich anfänglich bildenden homogenen Lösung schied sich das Produkt als Feststoff ab. Nach üblicher Isolierung erhielt man 0,6 g (14 % d.Th.) des gesuchten Calziumsalzes mit Fp. 175-178°C (Zers.).

Die in der nachfolgenden Tabelle 1 zusätzlich genannten Wirkstoffe können auf analogem Herstellungsweg erhalten werden.

Tabelle 1

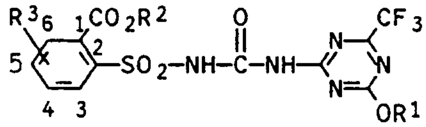

I

| Wirkstoff Nr. | $R^1$ | $R^2$ | $R^3$ | Fp [°C] |
|---|---|---|---|---|
| 1 | $CH_3$ | $CH_3$ | H | 165-167 |
| 2 | $CH_2CH_3$ | $CH_3$ | H | 164-168 |
| 3 | $CH_2CH_3$ | $CH_3$ | 6-F | 173-175 |
| 4 | $CH_3$ | $CH_3$ | 5-Cl | 144-145 |
| 5 | $CH_3$ | $CH_3$ | 6-Cl | 153-155 |
| 6 | $CH_3$ | $CH_3$ | 6-F | 166-168 |
| 7 | $CH_3$ | $CH_3$ | 4-Cl | 148-150 |
| 8 | $CH_3$ | $CH_3$ | 4-F | 153-157 |
| 9 | $CH_3$ | $CH_3$ | H | 175 (Na-Salz) |
| 10 | $CH_3$ | $CH_3$ | H | 175-178 (Ca-Salz) |
| 11 | $CH_3$ | $C_2H_5$ | H | 170-172 |

Anwendungsbeispiele:

Die herbizide Wirkung der [[(1,3,5-Triazin-2-yl)aminocarbonyl]aminosulfonyl]benzoesäureester der Formel I auf das Wachstum der Testpflanzen wird durch folgende Gewächshausversuche gezeigt.

Als Kulturgefäße dienen Plastikblumentöpfe mit 300 $cm^3$ Inhalt und lehmigem Sand mit etwa 3,0 % Humus als Substrat. Die Samen der Testpflanzen werden nach Arten getrennt flach eingesät.

Zum Zwecke der Nachauflaufbehandlung werden entweder direkt gesäte oder in den gleichen Gefäßen aufgewachsene Pflanzen ausgewählt oder sie werden erst als Keimpflanzen getrennt angezogen und einige Tage vor der Behandlung in die Versuchsgefäße verpflanzt.

Je nach Wuchsform werden die Testpflanzen bei einer Wuchshöhe von 3 bis 15 cm dann mit den in Wasser als Verteilungsmittel suspendierten oder emulgierten Wirkstoffen, die durch fein verteilende Düsen gespritzt werden, behandelt. Die Aufwandmenge für die Nachauflaufbehandlung beträgt 0,06 kg/ha a.S. (aktive Substanz).

Die Versuchsgefäße werden im Gewächshaus aufgestellt, wobei für wärmeliebende Arten wärmere Bereiche (20 bis 35 °C) und für solche gemäßigter Klimate 10 bis 20 °C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet.

Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzen sich aus folgenden Arten zusammen:

| Lateinischer Name | Deutscher Name |
|---|---|
| Amaranthus retroflexus | Zurückgekrümmter Fuchsschwanz |
| Cyperus iria | - |
| Galium aparine | Klettenlabkraut |
| Ipomoea spp. | Prunkwindenarten |
| Polygonum persicaria | Flohknöterich |
| Triticum aestivum | Sommerweizen |
| Veronica spp. | Ehrenpreisarten |

Mit 0,06 kg/ha a.S. im Nachauflaufverfahren eingesetzt lassen sich mit den Beispielen 1 und 2 breitblättrige unerwünschte Pflanzen sehr gut bekämpfen, bei gleichzeitiger hervorragender Selektivität in der Kulturpflanze Weizen.

In den folgenden Tabellen 2 und 3 sind Ergebnisse aus biologischen Untersuchungen zusammengestellt, in denen die erfindungsgemäßen Wirkstoffe und bekannte Verbindungen verglichen wurden.

Als bekannte Vergleichsverbindungen dienten die aus der EP-A 7 687 bekannten Sulfonylharnstoffe A und B:

A 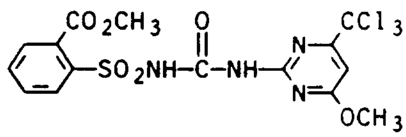

B 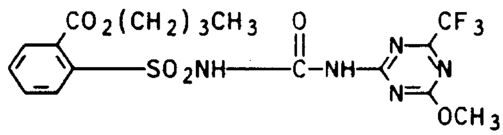

Tabelle 1

| Vergleich der herbiziden Aktivität der Beispielverbindung Nr. 1 mit der aus der EP-A 7 687 bekannten Vergleichsverbindung A bei Nachauflaufanwendung von 0,015 kg/ha a.S. im Gewächshaus | | |
|---|---|---|
| Testpflanzen | Schädigung [%] | |
| | Beispiel 1 | A |
| Triticum aestivum | 0 | 0 |
| Amaranthus retroflexus | 85 | 60 |
| Galium aparine | 98 | 10 |
| Ipomoea spp. | 80 | 50 |
| Polygonum persicaria | 80 | 30 |
| Veronica spp. | 100 | 20 |

Tabelle 2

| Vergleich der herbiziden Aktivität der Beispielsverbindung Nr. 2 mit der aus der EP-A 7 687 bekannten Vergleichsverbindung B bei Nachauflaufanwendung von 0,06 kg/ha a.S. im Gewächshaus | | |
|---|---|---|
| Testpflanzen | Schädigung [%] | |
| | Beispiel 2 | B |
| Triticum aestivum | 0 | 0 |
| Cyperus iria | 100 | 0 |
| Amaranthus retroflexus | 98 | 0 |
| Galium aparine | 95 | 0 |
| Ipomoea spp. | 100 | 10 |
| Polygonum persicaria | 100 | 0 |
| Veronica spp. | 100 | 0 |

**Patentansprüche**

1. [[(1,3,5-Triazin-2-yl)aminocarbonyl]aminosulfonyl]benzoesäureester der allgemeinen Formel I

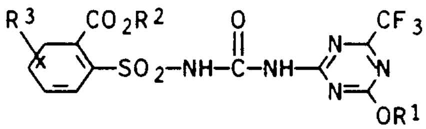

I

in der $R^1$ und $R^2$ eine Methyl- oder Ethylgruppe und $R^3$ Wasserstoff, Fluor oder Chlor bedeutet, sowie deren landwirtschaftlich brauchbare Salze.

2. Verfahren zur Herstellung der [[(1,3,5-Triazin-2-yl)aminocarbonyl]aminosulfonyl]benzoesäureester der Formel I gemäß Anspruch 1, dadurch gekennzeichnet, daß man ein Sulfonylisocyanat II

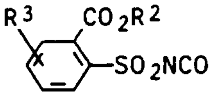

II

in an sich bekannter Weise in einem inerten organischen Lösungsmittel mit einem Über- oder Unterschuß von 0 bis 20 mol-% eines 2-Amino-1,3,5-triazin-derivats III

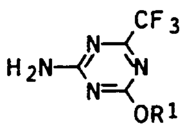

III

umsetzt.

3. Verfahren zur Herstellung der [[(1,3,5-Triazin-2-yl)aminocarbonyl]aminosulfonyl]benzoesäureester der Formel I gemäß Anspruch 1, dadurch gekennzeichnet, daß man ein Carbamat der Formel IV

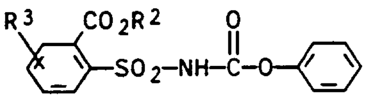

IV

in an sich bekannter Weise in einem inerten organischen Lösungsmittel bei einer Temperatur zwischen 0 und 120 °C mit einem Über- oder Unterschuß von 0 bis 20 mol-% eines 2-Amino-1,3,5-triazins III umsetzt.

4. Verfahren zur Herstellung der [[(1,3,5-Triazin-2-yl)aminocarbonyl]aminosulfonyl]benzoesäureester der Formel I gemäß Anspruch 1, dadurch gekennzeichnet, daß man ein entsprechendes Sulfonamid der Formel V

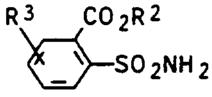

V

in an sich bekannter Weise in einem inerten organischen Lösungsmittel mit einem Über- oder Unterschuß von 0 bis 20 mol-% eines Phenylcarbamats VI

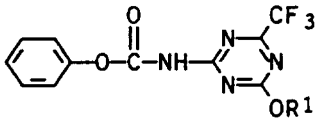

VI

umsetzt.

**5.** Verfahren zur Herstellung von Salzen der Verbindungen I gemäß Anspruch 1, dadurch gekennzeichnet, daß man eine Verbindung der Formel I gemäß Anspruch 1 in an sich bekannter Weise in Wasser oder einem inerten organischen Lösungsmittel mit einer Base deprotoniert.

**6.** Verfahren zur Herstellung der [[(1,3,5-Triazin-2-yl)-aminocarbonyl]aminosulfonyl]benzoesäureester der Formel I gemäß Anspruch 1, dadurch gekennzeichnet, daß man ein entsprechendes Sulfonamid der Formel V

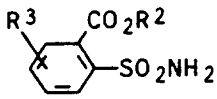

V

in an sich bekannter Weise in einem inerten organischen Lösungsmittel mit einem Isocyanat der Formel VII

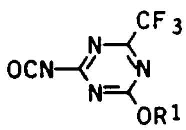

VII

umsetzt.

**7.** Herbizides Mittel, enthaltend einen [[(1,3,5-Triazin-2-yl)aminocarbonyl]aminosulfonyl]benzoesäureester der Formel I gemäß Anspruch 1 oder sein Salz sowie hierfür übliche Trägerstoffe.

**8.** Verfahren zur Bekämpfung unerwünschten Pflanzenwuchses, dadurch gekennzeichnet, daß man eine herbizid wirksame Menge eines [[(1,3,5-Triazin-2-yl)aminocarbonyl]aminosulfonyl]benzoesäureesters der Formel I gemäß Anspruch 1 oder eines seiner Salze auf die Pflanzen und/oder ihren Lebensraum einwirken läßt.

**9.** Verwendung der Verbindungen I gemäß Anspruch 1 als herbizide Mittel.

**Claims**

**1.** [[(1,3,5-Triazin-2-yl)aminocarbonyl]aminosulfonyl]benzoic esters of the general formula I

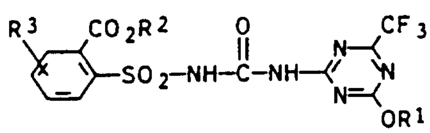

I

where $R^1$ and $R^2$ are methyl or ethyl, and $R^3$ is hydrogen, fluorine or chlorine, and agriculturally useful salts thereof.

**2.** A process for the preparation of [[(1,3,5-triazin-2-yl)aminocarbonyl]aminosulfonyl]benzoic esters of the formula I as claimed in claim 1, wherein a sulfonyl isocyanate II

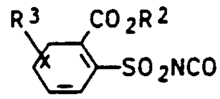

II

is reacted in conventional manner in an inert organic solvent with an amount which is greater or less than the stoichiometric amount by from 0 to 20 mol% of a 2-amino-1,3,5-triazine derivative III

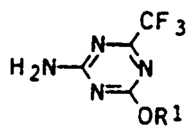

III

**3.** A process for the preparation of [[(1,3,5-triazin-2-yl)aminocarbonyl]aminosulfonyl]benzoic esters of the formula I as claimed in claim 1, wherein a carbamate of the formula IV

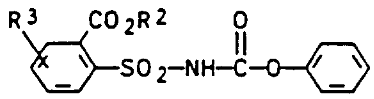

IV

is reacted in conventional manner in an inert organic solvent at from 0 to 120 °C with an amount which is greater or less than the stoichiometric amount by from 0 to 20 mol% of a 2-amino-1,3,5-triazine III as claimed in claim 2.

**4.** A process for the preparation of [[(1,3,5-triazin-2-yl)aminocarbonyl]aminosulfonyl]benzoic esters of the formula I as claimed in claim 1, wherein a corresponding sulfonamide of the formula V

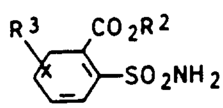

V

is reacted in conventional manner in an inert organic solvent with an amount which is greater or less than the stoichiometric amount by from 0 to 20 mol% of a phenyl carbamate VI

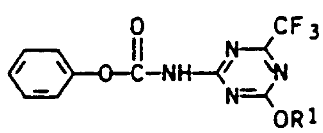

VI

**5.** A process for the preparation of salts of compounds I as set forth in claim 1, wherein a compound of the formula I as claimed in claim 1 is deprotonated with a base in conventional manner in water or an inert organic solvent.

**6.** A process for the preparation of [[(1,3,5-triazin-2-yl)aminocarbonyl]aminosulfonyl]benzoic esters of the formula I as claimed in claim 1, wherein a corresponding sulfonamide of the formula V

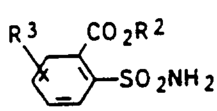

V

is reacted in conventional manner in an inert organic solvent with an isocyanate of the formula VII

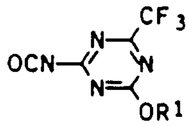

VII

7. A herbicidal agent containing a [[(1,3,5-triazin-2-yl)aminocarbonyl]aminosulfonyl]benzoic ester of the formula I as claimed in claim 1, or a salt thereof, and conventional carriers therefor.

8. A method of controlling unwanted plant growth, wherein a herbicidally effective amount of a [[(1,3,5-triazin-2-yl)aminocarbonyl]aminosulfonyl]benzoic ester of the formula I as claimed in claim 1, or a salt thereof, is allowed to act on the plants and/or their habitat.

9. The use of the compounds I as claimed in claim 1 as herbicidal agents.

**Revendications**

1. Esters [[(1,3,5-triazine-2-yl)-aminocarbonyl]-aminosulfonyl]-benzoïques de formule générale I

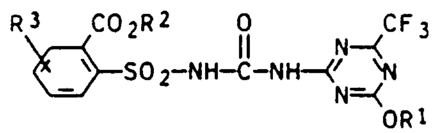

I

dans laquelle R1 et R2 représentent un groupe méthyle ou éthyle et R3 représente l'hydrogène, le fluor ou le chlore, et leurs sels convenant à l'utilisation en agriculture.

2. Procédé de préparation des esters [[(1,3,5-triazine-2-yl)-aminocarbonyl]-aminosulfonyl]-benzoïques de formule I de la revendication 1, caractérisé en ce que l'on fait réagir un sulfonylisocyanate II

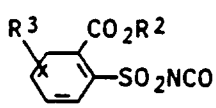

II

de manière connue en soi, dans un solvant organique inerte, avec un excès ou un défaut de 0 à 20 mol % d'un dérivé de la 2-amino-1,3,5-triazine III

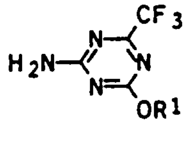

III

3. Procédé de préparation des esters [[(1,3,5-triazine-2-yl)-aminocarbonyl]-aminosulfonyl]-benzoïques de formule I de la revendication 1, caractérisé en ce que l'on fait réagir un carbamate de formule IV

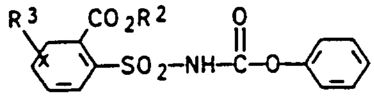

IV

de manière connue en soi, dans un solvant organique inerte, à une température de 0 à 120 °C, avec un excès ou un défaut de 0 à 20 mol % d'une 2-amino-1,3,5-triazine III.

**4.** Procédé de préparation des esters [[(1,3,5-triazine-2-yl)-aminocarbonyl]-aminosulfonyl]-benzoïques de formule I de la revendication 1, caractérisé en ce que l'on fait réagir un sulfonamide correspondant de formule V

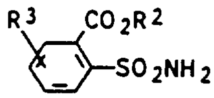

V

de manière connue en soi, dans un solvant organique inerte, avec un excès ou un défaut de 0 à 20 mol % d'un phénylcarbamate VI

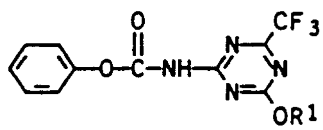

VI

**5.** Procédé de préparation des sels des composés I de la revendication 1, caractérisé en ce que l'on soumet un composé de formule I de la revendication 1 à déprotonisation de manière connue en soi, à l'aide d'une base, dans l'eau ou un solvant organique inerte.

**6.** Procédé de préparation des esters [[(1,3,5-triazine-2-yl)-aminocarbonyl]-aminosulfonyl]-benzoïques de formule I de la revendication 1, caractérisé en ce que l'on fait réagir un sulfonamide correspondant de formule V

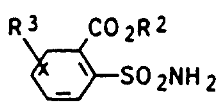

V

de manière connue en soi, dans un solvant organique inerte, avec un isocyanate de formule VII

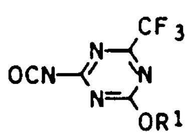

VII

**7.** Produit herbicide contenant un ester [[(1,3,5-triazine-2-yl)-aminocarbonyl]-aminosulfonyl]-benzoïque de formule I de la revendication 1 ou l'un de ses sels avec des véhicules usuels à cet effet.

**8.** Procédé pour combattre les croissances de végétaux indésirables , caractérisé en ce que l'on fait agir sur les végétaux et/ou leur habitat une quantité herbicide efficace d'un ester [[(1,3,5-triazine-2-yl)-aminocarbonyl]-aminosulfonyl]-benzoïque de formule I de la revendication 1 ou de l'un de ses sels.

**9.** Utilisation des composés I de la revendication 1 en tant que produits herbicides.